# DEMANDE DE BREVET EUROPEEN

(11) **EP 3 831 959 A1**
(43) Date de publication de la demande: **09.06.2021**
(21) Numéro de dépôt: 20211215.7
(22) Date de dépôt: 02.12.2020
(51) Int. Cl.: C12Q 1/6804, C12Q 1/6806, C12Q 1/6895, G01N 33/53

(54) **PROCÉDÉ DE DÉTECTION D'UNE ESPÈCE ALLERGÈNE, CONTAMINANTE OU DE NATURE FRAUDULEUSE DANS UN ÉCHANTILLON AGRO-ALIMENTAIRE**

(30) Priorité: 04.12.2019 FR 1913717
(71) Demandeur: Commissariat à l'Energie Atomique et aux Energies Alternatives, 75015 Paris (FR)
(72) Inventeur: CUBIZOLLES, Myriam-Laure, 38054 GRENOBLE Cedex 09 (FR); BOURDAT, Anne-Gaëlle, 38054 GRENOBLE cedex 09 (FR)
(74) Mandataire: INNOV-GROUP

(57) **Abrégé**

L'invention concerne un procédé de détection d'une espèce cible, de type allergène, contaminante ou de nature frauduleuse dans un échantillon agro-alimentaire (ECH), ledit procédé comportant :
- Une étape d'attaque chimique (E2) par mélange dudit échantillon agro-alimentaire (ECH) avec une solution corrosive (S_corr) en vue d'obtenir un extrait (EX) en solution ;
- Une première analyse (A1) comportant une étape de détection (E41) de l'ADN de l'espèce cible présente dans ledit extrait par séquençage ou analyse biomoléculaire, **et/ou**
- Une deuxième analyse (A2) comportant une étape de détection (E51) d'une protéine issue de l'espèce cible dans ledit extrait par analyse immunologique ou spectrométrique.

## Description

### Domaine technique de l'invention

La présente invention se rapporte à un procédé de détection d'une espèce allergène, contaminante ou de nature frauduleuse dans un échantillon agro-alimentaire.

### Etat de la technique

La présence d'espèces allergènes, contaminantes ou de nature frauduleuse dans un échantillon agro-alimentaire est un sujet sensible.

A titre d'exemple, les groupes d'aliments allergènes sont par exemple le gluten, le soja, le sésame, l'amande, la noisette,...

A titre d'exemple, les espèces contaminantes peuvent être les mycotoxines, les Bactéries, les virus, les cellules, les pesticides, les Antibiotiques, les perturbateurs endocriniens.

A titre d'exemple, les espèces de nature frauduleuse sont des composés supposés non présents dans l'aliment comme la poudre d'amande dans de la farine, de la viande de cheval mélangée avec de la viande de bœuf, des épices de curry dans du safran.

Le secteur agro-agro-alimentaire a aujourd'hui besoin de disposer de tests fiables, robustes, automatisés, rapides, faciles à mettre en œuvre et réalisables directement sur les sites de production. De même, pour leur permettre de détecter la présence d'espèces allergènes ou contaminantes dans leurs aliments, les utilisateurs doivent pouvoir disposer de dispositifs portables performants mettant en œuvre un test fiable et rapide.

Les espèces allergènes ou contaminantes agro-alimentaires :
- Sont pour l'essentiel des protéines ou des petites molécules parfois dégradées par les procédés de fabrication/transformation ;
- Sont insérées dans des matrices complexes ;
- Evoluent au cours de la durée de vie du produit et de son éventuelle transformation (cuisson, congélation,...) ;
- Peuvent être introduites fortuitement en faible quantité ;

Pour rappel, schématiquement, une molécule d'ADN va être transcrite en ARN qui sera ensuite traduit en protéine. En théorie, le même type d'information se trouve à la fois sur l'ADN initial et sur la protéine. Cependant, pour différentes raisons, ce n'est pas toujours le cas :
- Il existe une multitude de transcrits possibles à partir d'un même ADN ;
- Il existe une multitude de traductions possible en protéine à partir d'un ARN ;
- Les processus de fabrication (chauffage, rôtissage, congélation, exposition, mélange à d'autres substances...) sont susceptibles de modifier les protéines, ces processus pouvant supprimer un pouvoir allergène ou au contraire révéler et rendre accessible une zone allergène de la protéine ;

La mise en œuvre d'une méthode de détection fiable et rapide peut donc s'avérer plus complexe qu'on ne pourrait le penser.

Beaucoup de procédés et dispositifs ont déjà été proposés dans l'état de la technique. C'est le cas dans la demande de brevet WO2017/059103A1**,** WO2008/110655A1**,** US2016/266083A1 et le brevet US7585529B2**.**

Certains de ces documents antérieurs proposent des solutions de détection d'espèces allergènes par analyse biomoléculaire, d'autres des solutions de détection d'espèces allergènes par analyse immunologique.

Les méthodes de détection par analyse biomoléculaire présentent globalement les inconvénients suivants :
- Elles sont uniquement dédiées à l'analyse biomoléculaire ;
- Elles peuvent être toxiques ;
- Elles doivent être mises en œuvre à température plus élevée que la température ambiante ;
- Elles nécessitent une centrifugeuse et un appareillage spécifique de thermocyclage, chauffage et détection de fluorescence, ce qui les rendent difficiles à mettre en œuvre sur le terrain ;
- Elles nécessitent une étape de broyage de l'échantillon prélevé, entraînant une perte potentielle de sensibilité à la détection, par dilution des traces dans un large broyat ;

Les méthodes de détection par analyse immunologique présentent souvent les inconvénients suivants :
- Elles sont uniquement dédiées à l'analyse immunologique ;
- Elles peuvent être toxiques, notamment du fait de l'utilisation classique du 2β-mercaptoéthanol connu pour ses effets CMR (cancérogène, mutagène et toxique pour la reproduction) ;
- Elles peuvent être longues ;
- Elles peuvent nécessiter des manipulations sous hotte et l'emploi d'une centrifugeuse ;
- Elles peuvent occasionner une inactivation de l'anticorps ajouté pour la détection ;

Le but de l'invention est de proposer un procédé de détection d'une espèce allergène, contaminante ou de nature frauduleuse dans un échantillon agro-alimentaire qui puisse résoudre un ou plusieurs des inconvénients listés ci-dessus et constatés dans les méthodes antérieures.

Le procédé de l'invention permettra notamment de réaliser une détection d'une espèce allergène, contaminante ou de nature frauduleuse dans un échantillon agro-alimentaire qui est fiable, simple et rapide à mettre en œuvre, notamment directement sur le terrain.

### Exposé de l'invention

Ce but est atteint par un procédé de détection d'une espèce cible, de type allergène, contaminante ou de nature frauduleuse dans un échantillon agro-alimentaire, ledit procédé comportant :
- Une étape d'attaque chimique par mélange dudit échantillon agro-alimentaire avec une solution corrosive en vue d'obtenir un extrait en solution ;
- Une première analyse comportant une étape de détection de l'ADN de l'espèce cible présente dans ledit extrait par séquençage ou analyse biomoléculaire, **et/ou**
- Une deuxième analyse comportant une étape de détection d'une protéine issue de l'espèce cible dans ledit extrait par analyse immunologique ou spectrométrique.

Selon une particularité, une première partie de l'extrait est employée pour l'étape de détection de l'ADN de l'espèce cible et une deuxième partie de l'extrait est employée pour l'étape de détection de la protéine issue de l'espèce cible.

Selon une autre particularité, le procédé peut comporter une étape de filtration dudit extrait présent en solution pour récupérer un lysat.

Selon une autre particularité, le procédé comporte une étape de prélèvement dudit échantillon agro-alimentaire et l'étape d'attaque chimique est mise en œuvre sur ledit échantillon agro-alimentaire sans étape intermédiaire de broyage mécanique dudit échantillon agro-alimentaire.

Selon une autre particularité, la première analyse comporte une étape de purification/extraction de l'ADN.

Selon une autre particularité, l'étape de détection de l'ADN de l'espèce cible comporte une mise en œuvre d'une méthode de type q-PCR, ou par amplification isotherme de type LAMP ou RPA.

Selon une autre particularité, la deuxième analyse comporte une étape de solubilisation et/ou dénaturation et/ou réduction des protéines présentes dans l'extrait obtenu après l'étape d'attaque chimique.

Selon une autre particularité, l'étape de détection d'une protéine issue de l'espèce cible est réalisée par test de type ELISA ou EIA.

Selon une autre particularité, l'échantillon agro-alimentaire est mélangé avec la solution corrosive dans un ratio d'un volume d'échantillon sur un volume de solution corrosive qui est compris entre 0.1 et 1000.

Selon une autre particularité, l'échantillon agro-alimentaire est mélangé avec la solution corrosive dans un ratio d'un volume d'échantillon sur un volume de solution corrosive qui est compris entre 1 et 100.

Selon une autre particularité, l'échantillon agro-alimentaire est mélangé avec la solution corrosive dans un ratio d'un volume d'échantillon sur un volume de solution corrosive qui est compris entre 1 et 10.

Selon une autre particularité, la solution corrosive comporte des agents chaotropiques, des agents acides, des agents basiques, des solutions oxydantes et/ou détergentes, et/ou des sels.

Selon une autre particularité, la solution corrosive comporte un ou plusieurs des composants de la liste suivante :
- Urée,
- Sels de guanidine,
- Perchlorate ou hypochlorite de sodium,
- Potasse,
- Soude,
- Acide sulfurique, acide chlorhydrique, acide nitrique,
- Eau Oxygénée,
- Détergent ou Agent tensioactif (Tween, Triton, SDS),
- Chlorure de Sodium ou de Potassium.

L'invention concerne également un dispositif de détection d'une espèce cible, de type allergène, contaminante ou de nature frauduleuse dans un échantillon agro-alimentaire, ledit dispositif étant apte à mettre en œuvre le procédé tel que défini ci-dessus et comportant un boîtier logeant un réceptacle destiné à recevoir l'échantillon agro-alimentaire prélevé, un conteneur hermétique destiné à recevoir la solution corrosive, une interface homme machine et une unité centrale dotée de modules logiciels configurés pour :
- Commander un mélange de la solution corrosive avec l'échantillon agro-alimentaire prélevé ;
- Commander une exécution de la première analyse par détection de l'ADN de l'espèce cible sélectionnée ; et/ou
- Commander une exécution de la deuxième analyse par détection de la protéine de l'espèce cible sélectionnée ;
- Récupérer et analyser des premières mesures effectuées lors de la première analyse ;
- Récupérer et analyser des deuxièmes mesures effectuées lors de la deuxième analyse ;
- Déterminer la présence l'espèce cible en fonction des premières mesures et deuxièmes mesures récupérées et analysées ;
- Commander l'interface homme-machine pour afficher un résultat d'analyse ;

Il faut noter qu'il peut s'avérer particulièrement pertinent de mettre en œuvre les deux types d'analyse en parallèle et que c'est encore plus intéressant quand cela est réalisé à partir d'un même échantillon de départ.

Il faut rappeler que :
- La présence de l'ADN de l'espèce allergène indique à coup sûr la présence de l'espèce. Néanmoins, les protéines issues de cet ADN peuvent potentiellement ne pas être allergènes, ou encore ne pas exister du tout (pas de traduction et/ou pas de transcription).
- La détection de présence de protéines d'une espèce allergène ne permet de cibler que quelques zones particulières qui correspondent aux épitopes de la protéine reconnus par les anticorps qui permettent le dosage. Autrement dit, une protéine peut très bien porter un caractère allergène mais celui-ci ne sera pas forcément détecté si aucun anticorps ciblant ces zones allergéniques n'est disponible.

A titre d'exemple, pour l'espèce allergène de type gluten :
- Une analyse PCR permet de quantifier l'ADN du gluten, ou plus exactement les ADN de blé, orge, seigle et avoine.
- Un test immunologique de type ELISA permet de doser les prolamines, protéines constitutives du gluten (gliadine pour le blé, sécaline pour le seigle, hordéine pour l'orge) contenant les séquences peptidiques susceptibles de déclencher la réaction allergique chez les personnes.

Les deux approches permettent donc de corréler les données obtenues après l'analyse PCR et le test ELISA et de comparer les différences potentielles de quantification entre ces deux approches.

Même si dans certains cas, une seule analyse peut s'avérer suffisante, la mise en œuvre des deux analyses en parallèle peut donc s'avérer particulièrement pertinente.

### Brève description des figures

D'autres caractéristiques et avantages vont apparaître dans la description détaillée qui suit faite en regard des dessins annexés dans lesquels :
- La figure 1 représente de manière schématique le principe du procédé de l'invention ;
- La figure 2 illustre le principe d'attaque chimique réalisée sur un échantillon agro-alimentaire hétérogène tel que des graines ;
- La figure 3 représente de manière schématique l'architecture d'un dispositif capable de mettre en œuvre le procédé de l'invention ;

### Description détaillée d'au moins un mode de réalisation

Le procédé de détection d'une espèce allergène, d'une espèce contaminante ou de nature frauduleuse est mis en œuvre sur un échantillon agro-alimentaire ECH.

Dans la suite de la description, nous ferons référence à l'espèce cible pour évoquer l'espèce allergène, l'espèce contaminante ou l'espèce de nature frauduleuse recherchée.

Cet échantillon agro-alimentaire ECH peut être de tous types, par exemple des graines, de la farine, une pâte (par exemple pâte à tartiner) ou être formé de tout prélèvement d'une matrice agro-alimentaire réalisée sur une chaîne de fabrication ou directement par le consommateur final lors d'un repas (ex : produit fini tel que sauce bolognaise, cassoulet, etc...). On verra que le procédé de l'invention présente un intérêt particulier lorsque la matrice agro-alimentaire est hétérogène (graines, morceaux...).

Le procédé de l'invention consiste notamment à mettre en œuvre une étape d'obtention d'un extrait en solution qui peut être employé à la fois pour :
- Une première analyse par détection de l'ADN de l'espèce cible présente dans ledit extrait, par séquençage ou analyse biomoléculaire, et/ou
- Une deuxième analyse par détection d'une protéine issue de l'espèce cible dans ledit extrait, par analyse immunologique ou spectrométrique.

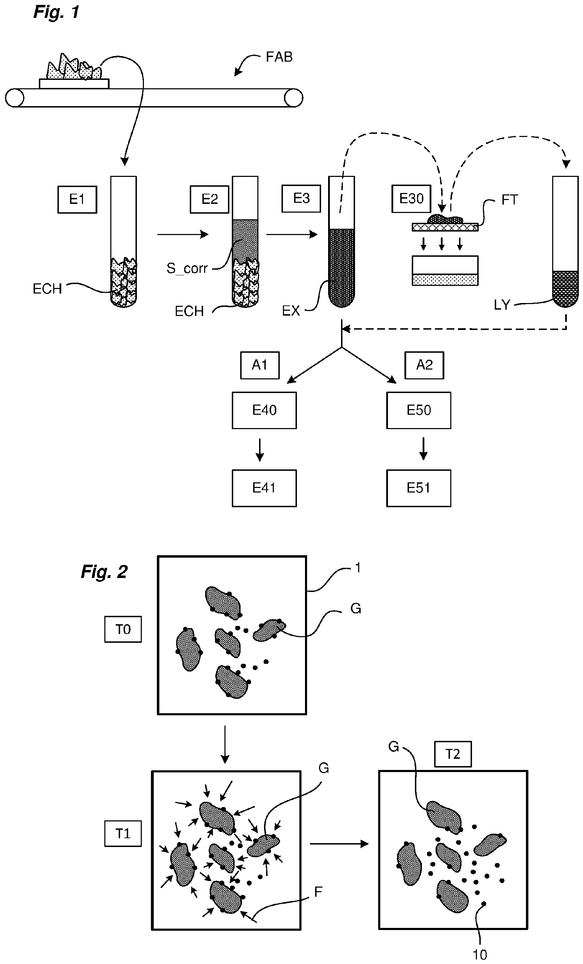

En référence à la figure 1, après une étape de prélèvement E1 de l'échantillon agro-alimentaire ECH (par exemple sur une chaîne de fabrication FAB), le procédé comporte ainsi une étape de préparation E2 commune aux deux types d'analyses A1, A2 qui peuvent être effectuées ultérieurement, que ce soit par détection de présence de l'ADN de l'espèce cible ou par détection de présence d'une protéine issue de l'espèce cible.

L'étape de préparation E2 consiste en une attaque chimique de l'échantillon agro-alimentaire ECH prélevé, par mélange dudit échantillon agro-alimentaire avec une solution corrosive S_corr en vue d'obtenir un extrait EX en solution.

A titre d'exemple, la solution corrosive S_corr peut comporter des agents chaotropiques, des agents acides, des agents basiques, des solutions oxydantes et/ou détergentes, et/ou des sels

La solution corrosive peut comporter un ou plusieurs des composants suivants :
- Urée,
- Sels de guanidine,
- Perchlorate ou hypochlorite de sodium,
- Potasse,
- Soude,
- Acide sulfurique, acide chlorhydrique, acide nitrique,
- Eau Oxygénée,
- Détergent ou Agent tensioactif (Tween, Triton, SDS),
- Chlorure de Sodium ou de Potassium.

Selon une particularité, l'échantillon agro-alimentaire ECH est mélangé avec la solution corrosive S_corr dans un ratio d'un volume d'échantillon sur un volume de solution corrosive qui est compris entre 0.1 et 1000, préférentiellement compris entre 1 et 100 et préférentiellement compris entre 1 et 10, idéalement égal à 4.

Idéalement un volume d'échantillon est donc dilué dans quatre volumes de solution corrosive.

Dans certains cas, les quantités à détecter sont très faibles dans l'échantillon agro-alimentaire ECH prélevé et peuvent être présentes simplement à l'état de traces. Le fait de réaliser une attaque chimique sur l'échantillon agro-alimentaire permet de récupérer les poussières et de surreprésenter les traces de l'espèce cible présente dans l'échantillon agro-alimentaire.

Ceci est particulièrement efficace lorsque l'échantillon agro-alimentaire ECH est de type hétérogène, par exemple composé de graines ou de morceaux. Dans ce cas particulier, l'attaque chimique va permettre d'attaquer les graines ou morceaux en surface pour détacher les traces de l'espèce cible et ainsi les rendre plus facilement détectables. Ce principe est notamment illustré par la figure 2.

Sur cette figure 2
A T0 : les graines G sont prélevées dans un réceptacle 1 ;
A T1 : La solution corrosive est ajoutée. Les flèches F illustrent le principe d'attaque chimique sur les graines G.
A T2 : Grâce à l'attaque chimique, les traces 10 et poussières sont détachées de la surface des graines G permettant une surreprésentation.

Selon un aspect particulier de l'invention, lorsque l'échantillon agro-alimentaire ECH prélevé est dans un état hétérogène, il n'est donc nul besoin de le broyer avant l'étape de préparation E2 par attaque chimique ce qui permet de gagner du temps sur l'ensemble du process d'analyse tout en améliorant l'efficacité de la détection.

Après l'étape de préparation E2, on obtient un extrait en solution (EX - E3).

Cet extrait en solution EX peut ensuite être employé à la fois pour la première analyse A1 par détection de l'ADN de l'espèce cible et pour la deuxième analyse A2 par détection d'une protéine de l'espèce cible.

Selon un principe de l'invention, il est en effet possible de mettre en œuvre les deux types d'analyse en parallèle à partir d'un même extrait obtenu après l'étape d'attaque chimique. Bien entendu, il sera également possible de mettre en œuvre l'un ou l'autre des deux types d'analyse.

L'analyse de présence de l'ADN de l'espèce cible peut ensuite être réalisée par toute solution connue. Elle peut comporter une étape d'extraction/purification E40 des molécules d'ADN présentes dans l'extrait de solution obtenu. Cette étape d'extraction/purification peut par exemple consister en une précipitation de l'ADN présent dans l'extrait de solution. Cette étape de précipitation peut être réalisée par toute méthode connue, par exemple précipitation alcoolique, au froid ou en présence de sels.

Une fois l'ADN extrait, l'étape de détection E41 de l'ADN de l'espèce cible peut être réalisée par séquençage ou par analyse biomoléculaire. De manière non limitative, l'analyse biomoléculaire peut être mise en œuvre selon des techniques classiques de type qPCR ("Quantitive Polymerase Chain Reaction") ou par amplification isotherme de type LAMP (Loop mediated isothermal Amplification) ou RPA ("Recombinase Polymerase Amplification").

L'analyse de la présence de protéine de l'espèce cible peut également être réalisée par toute solution connue. De manière non limitative, elle peut comporter une étape E50 préalable de solubilisation et/ou dénaturation et/ou réduction des protéines présentes dans l'extrait EX obtenu après l'étape d'attaque chimique. Cette étape E50 peut être réalisée par toute méthode connue, par ajout d'un alcool (éthanol), par ajout d'un agent chaotropique (par exemple Guanidine), par ajout d'un agent réducteur (par exemple le Dithiothréitol DTT).

Par la suite, après extraction des protéines de l'espèce cible, l'étape de détection E51 peut être réalisée par analyse immunologique ou spectrométrique. L'analyse immunologique peut consister en un test immunologique classique de type ELISA ou EIA.

De manière optionnelle, l'extrait en solution EX qui est obtenu à l'étape E3 peut être filtré lors d'une étape de filtration E30 à travers un filtre FT pour ne conserver qu'un lysat LY qui pourra ensuite être employé pour une première partie pour la première analyse A1 par détection de l'ADN de l'espèce cible et pour une deuxième partie pour la deuxième analyse par détection de la protéine de l'espèce cible selon les deux principes évoqués ci-dessus.

Pour valider la pertinence du procédé de l'invention, une première expérience a été menée. Un échantillon agro-alimentaire garanti sans gluten est dopé en gluten de deux façons différentes :
- Ajout d'un grain de blé dans un échantillon de 3g de graines qui est garanti sans gluten ;
- Ajout d'un grain de blé dans un échantillon de 3 g de graines qui est garanti sans gluten, agitation manuelle et retrait du grain de blé avec une pince ;

Les deux échantillons sont ensuite mélangés avec la solution corrosive S_corr selon le procédé de l'invention. En effectuant ensuite une détection d'ADN du gluten par q-PCR, la présence de gluten est détectée dans les deux échantillons, même dans celui où le grain de blé a été retiré. Les traces sont donc détectables, grâce à l'action chimique réalisée pour surreprésenter les traces.

On comprend de ce qui précède que le procédé de l'invention présente les avantages suivants :
- Niveau de sensibilité accru, obtenu notamment par l'absence de broyage de l'échantillon agro-alimentaire ECH prélevé et grâce à la mise en œuvre de l'attaque chimique qui permet de détacher les poussières et donc d'obtenir une surreprésentation des traces présentes ;
- Solution rapide à mettre en œuvre : La préparation de l'extrait EX à analyser est commune aux deux analyses A1, A2 ultérieures, détection de présence de l'ADN ou détection de protéines de l'ADN. De plus, il n'est pas nécessaire de prévoir un délai entre l'étape de prélèvement de l'échantillon ECH et l'étape de préparation E2, ce qui permet d'éviter toute dégradation des échantillons.
- Solution facile à mettre en œuvre, pouvant être réalisée sur le terrain (chaîne de fabrication) ou par un utilisateur à l'aide d'un dispositif adapté.
- Procédé compatible avec tous les types d'échantillon agro-alimentaire (graines, pâte, farine, etc...), même si plus favorable aux matrices hétérogènes par rapport aux méthodes conventionnelles existantes.
- Solution compatible avec le dosage de n'importe quelle espèce allergène (gluten, soja, sésame, amande, noisette, etc...), espèce contaminante (par exemple : mycotoxine, Bactéries, virus, cellules, Pesticides, Antibiotiques, Perturbateurs endocriniens) ou de nature frauduleuse (poudre d'amande avec farine ou autres graines, viande de cheval dans viande de bœuf, mélange d'épices curry dans du safran...)
- Solution compatible avec les méthodes de détections ADN, de type q-PCR, amplifications isothermes (LAMP, RPA...) et séquençage et avec les méthodes de détection par analyse immunologique (EIA, ELISA).
- Solution permettant de réaliser les deux types d'analyse (biologie moléculaire et immunologique) à partir d'un seul et même échantillon, donc pas de problème de représentativité, puisque les deux mesures seront obtenues sur le même prélèvement.

Le procédé peut notamment être mis en œuvre dans un dispositif adapté, avantageusement portable et facile à utiliser que ce soit sur le terrain ou par un utilisateur qui souhaite effectuer des tests à titre personnel.

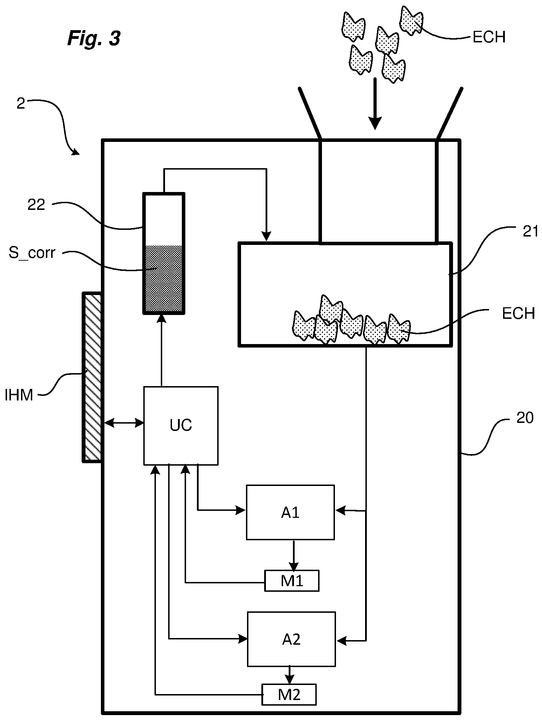

En référence à la figure 3, ce dispositif 2 peut présenter l'architecture simplifiée suivante :
- Un boîtier 20 externe ;
- Un réceptacle 21 destiné à recevoir l'échantillon agro-alimentaire prélevé ;
- Un conteneur 22 stockant de manière hermétique la solution corrosive S_corr ;
- Une interface homme-machine IHM de type boutons et ou écran pour commander le dispositif 2, agencé sur le boîtier ; L'interface homme-machine pourra notamment permettre de sélectionner l'espèce cible recherchée.
- Une unité centrale UC comportant un microcontrôleur chargé d'exécuter des modules logiciels configurés pour mettre en œuvre le procédé de l'invention ;

L'unité centrale est chargée de :
- Recevoir des commandes en provenance de l'interface homme-machine pour lancer le processus de détection ;
- Commander le mélange de la solution corrosive avec l'échantillon agro-alimentaire ECH prélevé ;
- Commander l'exécution de la première analyse A1 par détection de l'ADN de l'espèce cible sélectionnée ; et/ou
- Commander l'exécution de la deuxième analyse A2 par détection de la protéine de l'espèce cible sélectionnée ;
- Récupérer et analyser les mesures M1 effectuées lors de la première analyse A1 ;
- Récupérer et analyser les mesures M2 effectuées lors de la deuxième analyse A2 ;
- Déterminer la présence l'espèce cible en fonction des mesures M1, M2 récupérées et analysées ;
- Commander l'interface homme-machine IHM pour communiquer un résultat d'analyse, par exemple via un affichage sur un écran ;

Des moyens mécaniques et pneumatiques sont avantageusement intégrés au dispositif pour permettre les transferts de matière lors du processus.

Des moyens optiques peuvent également être présents pour effectuer les analyses de type q-PCR par exemple.

## Revendications

1. Procédé de détection d'une espèce cible, de type allergène, contaminante ou de nature frauduleuse dans un échantillon agro-alimentaire (ECH), **caractérisé en ce qu'**il comporte :
- Une étape d'attaque chimique (E2) par mélange dudit échantillon agro-alimentaire (ECH) avec une solution corrosive (S_corr) en vue d'obtenir un extrait (EX) en solution ;
- Une première analyse (A1) comportant une étape de détection (E41) de l'ADN de l'espèce cible présente dans ledit extrait par séquençage ou analyse biomoléculaire, **et/ou**
- Une deuxième analyse (A2) comportant une étape de détection (E51) d'une protéine issue de l'espèce cible dans ledit extrait par analyse immunologique ou spectrométrique.

2. Procédé selon la revendication 1, **caractérisé en ce qu'**une première partie de l'extrait (EX) est employée pour l'étape de détection (E41) de l'ADN de l'espèce cible et **en ce qu'**une deuxième partie de l'extrait (EX) est employée pour l'étape de détection (E51) de la protéine issue de l'espèce cible.

3. Procédé selon la revendication 1 ou 2, **caractérisé en ce qu'**il comporte une étape de filtration (E30) dudit extrait présent en solution pour récupérer un lysat (LY).

4. Procédé selon l'une des revendications 1 à 3, **caractérisé en ce qu'**il comporte une étape de prélèvement (E1) dudit échantillon agro-alimentaire et **en ce que** l'étape d'attaque chimique (E2) est mise en œuvre sur ledit échantillon agro-alimentaire (ECH) sans étape intermédiaire de broyage mécanique dudit échantillon agro-alimentaire.

5. Procédé selon l'une des revendications 1 à 4, **caractérisé en ce que** la première analyse (A1) comporte une étape de purification/extraction (E40) de l'ADN.

6. Procédé selon l'une des revendications 1 à 5, **caractérisé en ce que** l'étape de détection (E41) de l'ADN de l'espèce cible comporte une mise en œuvre d'une méthode de type q-PCR, ou par amplification isotherme de type LAMP ou RPA.

7. Procédé selon l'une des revendications 1 à 6, **caractérisé en ce que** la deuxième analyse (A2) comporte une étape (E50) de solubilisation et/ou dénaturation et/ou réduction des protéines présentes dans l'extrait (EX) obtenu après l'étape d'attaque chimique (E2).

8. Procédé selon l'une des revendications 1 à 7, **caractérisé en ce que** l'étape de détection (E51) d'une protéine issue de l'espèce cible est réalisée par test de type ELISA ou EIA.

9. Procédé selon l'une des revendications 1 à 8, **caractérisé en ce que** l'échantillon agro-alimentaire (ECH) est mélangé avec la solution corrosive dans un ratio d'un volume d'échantillon sur un volume de solution corrosive qui est compris entre 0.1 et 1000.

10. Procédé selon l'une des revendications 1 à 8, **caractérisé en ce que** l'échantillon agro-alimentaire (ECH) est mélangé avec la solution corrosive dans un ratio d'un volume d'échantillon sur un volume de solution corrosive qui est compris entre 1 et 100.

11. Procédé selon l'une des revendications 1 à 8, **caractérisé en ce que** l'échantillon agro-alimentaire (ECH) est mélangé avec la solution corrosive dans un ratio d'un volume d'échantillon sur un volume de solution corrosive qui est compris entre 1 et 10.

12. Procédé selon l'une des revendications 1 à 9, **caractérisé en ce que** la solution corrosive (S_corr) comporte des agents chaotropiques, des agents acides, des agents basiques, des solutions oxydantes et/ou détergentes, et/ou des sels.

13. Procédé selon l'une des revendications 1 à 9, **caractérisé en ce que** la solution corrosive (S_corr) comporte un ou plusieurs des composants de la liste suivante :
- Urée,
- Sels de guanidine,
- Perchlorate ou hypochlorite de sodium,
- Potasse,
- Soude,
- Acide sulfurique, acide chlorhydrique, acide nitrique,
- Eau Oxygénée,
- Détergent ou Agent tensioactif (Tween, Triton, SDS),
- Chlorure de Sodium ou de Potassium.

14. Dispositif de détection d'une espèce cible, de type allergène, contaminante ou de nature frauduleuse dans un échantillon agro-alimentaire (ECH), **caractérisé en ce qu'**il est apte à mettre en œuvre le procédé tel que défini dans l'une des revendications 1 à 13 et **en ce qu'**il comporte un boîtier (20) logeant un réceptacle (21) destiné à recevoir l'échantillon agro-alimentaire (ECH) prélevé, un conteneur hermétique destiné à recevoir la solution corrosive (S_corr), une interface homme machine (IHM) et une unité centrale (UC) dotée de modules logiciels configurés pour :
- Commander un mélange de la solution corrosive avec l'échantillon agro-alimentaire (ECH) prélevé ;
- Commander une exécution de la première analyse (A1) par détection de l'ADN de l'espèce cible sélectionnée ; et/ou
- Commander une exécution de la deuxième analyse (A2) par détection de la protéine de l'espèce cible sélectionnée ;
- Récupérer et analyser des premières mesures (M1) effectuées lors de la première analyse (A1) ;
- Récupérer et analyser des deuxièmes mesures (M2) effectuées lors de la deuxième analyse (A2) ;
- Déterminer la présence l'espèce cible en fonction des premières mesures (M1) et deuxièmes mesures (M2) récupérées et analysées ;
- Commander l'interface homme-machine (IHM) pour afficher un résultat d'analyse ;
